# EUROPEAN PATENT SPECIFICATION

(11) **EP 4 646 999 B1**
(45) Date of publication and mention of the grant of the patent: **02.09.2026**
(21) Application number: 25162192.6
(22) Date of filing: 06.03.2025
(51) Int. Cl.: A61B 5/00

(54) **WEARABLE RING-SHAPED PENILE ERECTION HARDNESS MEASURING DEVICE AND METHOD FOR MEASUREING PENILE ERECTION HARDNESS**
AM KÖRPER TRAGBARE RINGFÖRMIGE VORRICHTUNG ZUR MESSUNG DER EREKTIONSHÄRTE EINES PENIS UND VERFAHREN ZUR MESSUNG DER EREKTIONSHÄRTE EINES PENIS
DISPOSITIF DE MESURE DE DURETÉ D'ÉRECTION PÉNIENNE EN FORME D'ANNEAU POUVANT ÊTRE PORTÉ ET PROCÉDÉ DE MESURE DE DURETÉ D'ÉRECTION PÉNIENNE

(30) Priority: 26.03.2024 TW 113111305
(43) Date of publication of application: 12.11.2025
(73) Proprietor: Lin, Yi-Ting, Huwei Township, Yunlin 63252 (TW)
(72) Inventor: Lin, Yi-Ting, Huwei Township, Yunlin 63252 (TW)
(74) Representative: Murgitroyd & Company

(56) References cited:
- WO-A2-2019/239277
- GB-A- 2 212 924
- KR-A- 20190 115 833
- US-A1- 2016 174 871
- US-A1- 2019 328 324

## Description

### CROSS REFERENCE TO RELATED APPLICATIONS

This application claims the priority of Taiwan Patent Application No. 113111305, filed on March 26, 2024, titled "WEARABLE RING-SHAPED PENILE ERECTION HARDNESS MEASURING DEVICE AND METHOD FOR MEASUREING PENILE ERECTION HARDNESS".

### FIELD OF INVENTION

The present application relates to measuring devices for physiological characteristics, and more particularly, to a wearable ring-shaped penile erection hardness measuring device and a method of measuring penile erection hardness.

### BACKGROUND OF INVENTION

Male sexual function is a comprehensive performance of male physical health. However, male sexual dysfunction may result from psychological or physiological disorders. Male sexual function includes three main indicators: penile erection hardness, penile erection duration, and intravaginal ejaculation latency time (IELT). Traditional measurement method uses various self-evaluation erectile function scales. However, this self-evaluation method can be affected by the following factors, thereby resulting some deviations: self-awareness, limited exposure to penises of various hardness levels (i.e. lack of comparison standard), and personal subjective factors.

In the existing technology, there are various penile hardness measuring devices to measure penile hardness during sleep. However, the atmosphere and situation of this kind of measurement are very different from actual sexual activity, and the results cannot reflect the actual penile hardness during sexual activity. To solve the above-mentioned problem, various wearable ring-shaped measuring devices have been developed to measure the actual hardness of the penis during sexual activity. For example, a metal ring is used to measure penile hardness, but the sizes of the penis of the subjects are different, and a metal ring with inappropriate size will cause discomfort to the subjects. Furthermore, some wearable ring-shaped measuring devices require complex components to perform measurements (for example, through motors), which will significantly increase the structural complexity, weight, and manufacturing cost of the wearable ring-shaped measuring device.

Therefore, it is necessary to provide a wearable ring-shaped penile erection hardness measuring device and a method of measuring penile erection hardness to solve the problems in the conventional technology. WO 2019/239277 A2 discloses a wearable ring-shaped penile erection hardness measuring device comprising an elastic ring-shaped body with a sensing portion having an outer layer and a non-sensing portion connected to the sensing portion.

### SUMMARY OF INVENTION

The present invention is directed to a wearable ring-shaped penile erection hardness measuring device according to claim 1 and corresponding method of measuring penile erection hardness according to claim 10. Additional aspects of the invention are defined in dependent claims.
An objective of the present disclosure is to provide a wearable ring-shaped penile erection hardness measuring device. The wearable ring-shaped penile erection hardness measuring device not only can measure penile hardness during actual sexual activity, but also can simplify the structure of the device, thereby reducing the assembly difficulty and the overall weight of the device.

Another objective of the present disclosure is to provide a method of measuring penile erection hardness, which can accurately present penile hardness of male by comparing it with standard products (for example, a variety of homogeneous silicone dildos with different Shore hardness values).

In order to achieve the above objectives, the present disclosure provides a wearable ring-shaped penile erection hardness measuring device for measuring a hardness value of a penis to be tested, the wearable ring-shaped penile erection hardness measuring device includes: an elastic ring-shaped body configured to be worn on the penis to be tested, the elastic ring-shaped body including: a sensing portion, the sensing portion comprising: an inner layer, an outer layer, a sensing protrusion, a thin film sensing element, and an accommodating space, wherein the accommodating space is located between the inner layer and the outer layer, the sensing protrusion is configured to contact the penis to be tested, the inner layer is connected to the sensing protrusion, the thin film sensing element is disposed in the accommodating space, the thin film sensing element contacts the inner layer, and the thin film sensing element is configured to measure an applying force value from the penis to be tested, wherein an outer layer thickness of the outer layer is greater than an inner layer thickness of the inner layer; and a non-sensing portion connected to the sensing portion; an electronic component accommodating portion connected to the elastic ring-shaped body; and a processing unit electrically connected to the thin film sensing element to convert the applying force value into the hardness value.

In one embodiment of the present disclosure, the sensing portion further includes a spacer disposed in the accommodating space, and the spacer is located between the thin film sensing element and the outer layer.

In one embodiment of the present disclosure, the outer layer thickness is between 1.5 mm and 3 mm, and the inner layer thickness is between 0.5 mm and 1.2 mm.

In one embodiment of the present disclosure, the thin film sensing element is a pressure sensitive element.

In one embodiment of the present disclosure, an inner diameter of the elastic ring-shaped body is between 2.2 mm and 5.2 mm.

In one embodiment of the present disclosure, the wearable ring-shaped penile erection hardness measuring device further includes: a light emitting element adjacent to the electronic component accommodating portion, wherein the light emitting element is configured to provide a light with a certain wavelength to the penis to be tested.

In one embodiment of the present disclosure, the elastic ring-shaped body is made of an elastic material.

In one embodiment of the present disclosure, a Shore hardness value of the elastic material is between 20 and 50.

In one embodiment of the present disclosure, the elastic material is made of a silicone.

The present disclosure further provides a method of measuring penile erection hardness for measuring a hardness value of a penis to be tested, the method of measuring penile erection hardness includes: wearing a wearable ring-shaped penile erection hardness measuring device on the penis to be tested; obtaining an applying force value from the penis to be tested, by the thin film sensing element of the wearable ring-shaped penile erection hardness measuring device; and comparing the applying force value with a plurality of simulated applying forces, by the processing unit of the wearable ring-shaped penile erection hardness measuring device, each of the simulated applying forces corresponding to a Shore hardness value, and in response to the applying force value matching one of the simulated applying forces, determining the Shore hardness value corresponding to the simulated applying force as the hardness value of the penis to be tested.

As described above, the present disclosure transmits the applying force from the penis to be tested to the thin film sensing element through the sensing protrusion, the inner layer, and the outer layer, such that the thin film sensing element can accurately obtain the applying force value from the penis to be tested, and then the applying force value can be converted into the hardness value of the penis to be tested by the processing unit. Measuring the applying force, by the thin film sensing element, can simplify the structure of the wearable ring-shaped penile erection hardness measuring device, thereby reducing the weight of the wearable ring-shaped penile erection hardness measuring device.

### DESCRIPTION OF DRAWINGS

In order to more clearly illustrate the technical solutions in the embodiments or the prior art, the following drawings, which are intended to be used in the description of the embodiments or the prior art, will be briefly described. It will be apparent that the drawings and the following description are only some embodiments of the present invention. Those of ordinary skill in the art may, without creative efforts, derive other drawings from these drawings.
FIG. 1 is a schematic front view of a wearable ring-shaped penile erection hardness measuring device according to an embodiment of the present disclosure.
FIG. 2 is a schematic cross-section view of the embodiment of FIG. 1.
FIG. 3 is a schematic perspective view of the embodiment of FIG. 1.
FIG. 4 is a schematic connection diagram of the electronic components in the embodiment of FIG. 1.
FIG. 5 shows the measurement principle of the embodiment of FIG. 1 at non-erection state.
FIG. 6 shows the measurement principle of the embodiment of the FIG. 1 at erection state.
FIG. 7 is a schematic diagram of the combination of the embodiment of the FIG. 1 and a charging device.
FIG. 8 is a flow chart of a method of measuring penile erection hardness according to an embodiment of the present disclosure.

### DETAILED DESCRIPTION OF PREFERRED EMBODIMENTS

In the following detailed description of the preferred embodiments, reference is made to the accompanying drawings which form a part hereof, and in which is shown by way of illustration specific embodiments in which the invention may be practiced. In this regard, directional terminology, such as "top", "bottom", "front", "back", "left", "right", "inside", "outside", "side", etc., is used with reference to the orientation of the figure(s) being described. As such, the directional terminology is used for purposes of illustration and is in no way limiting. Throughout this specification and in the drawings like parts will be referred to by the same reference numerals.

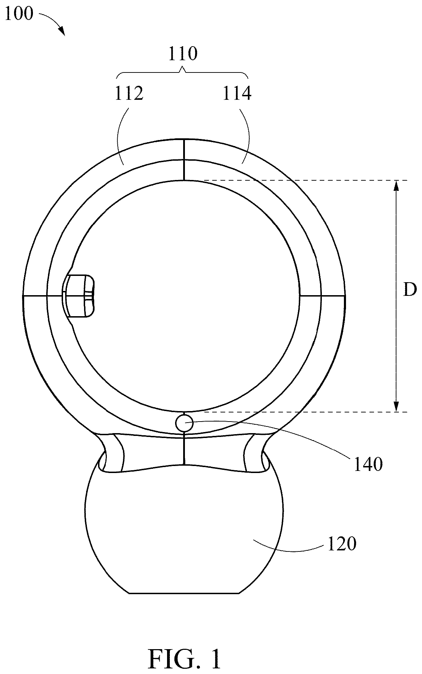

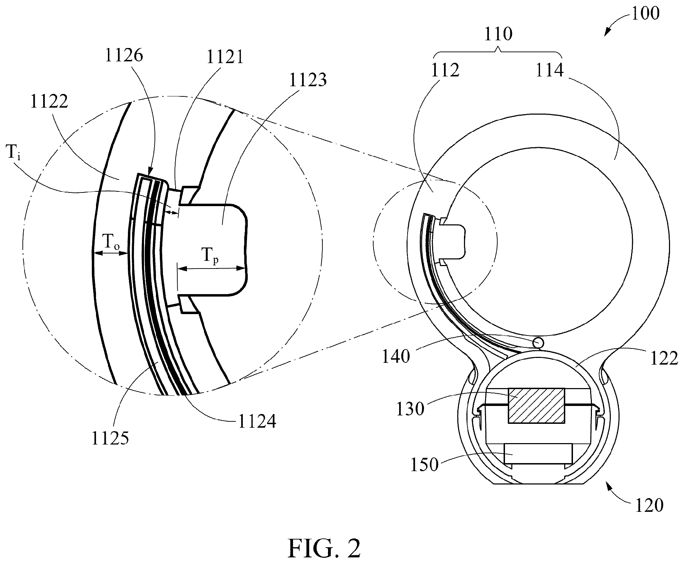

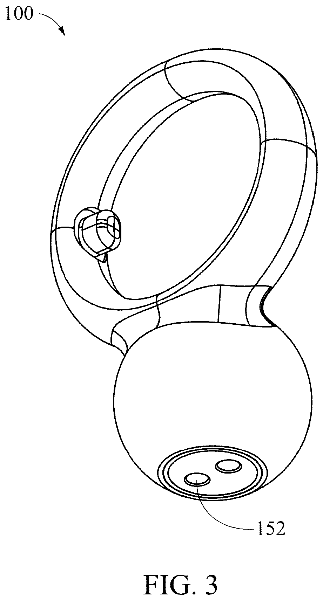

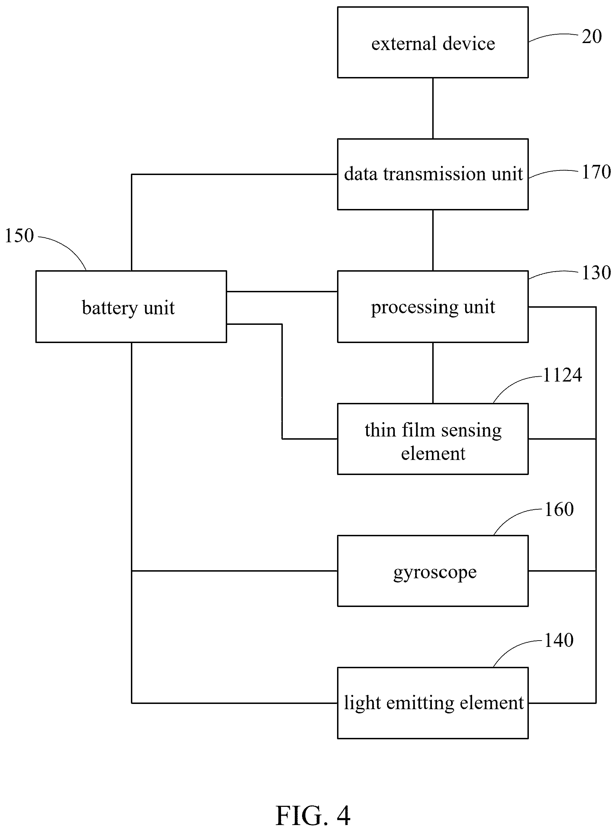

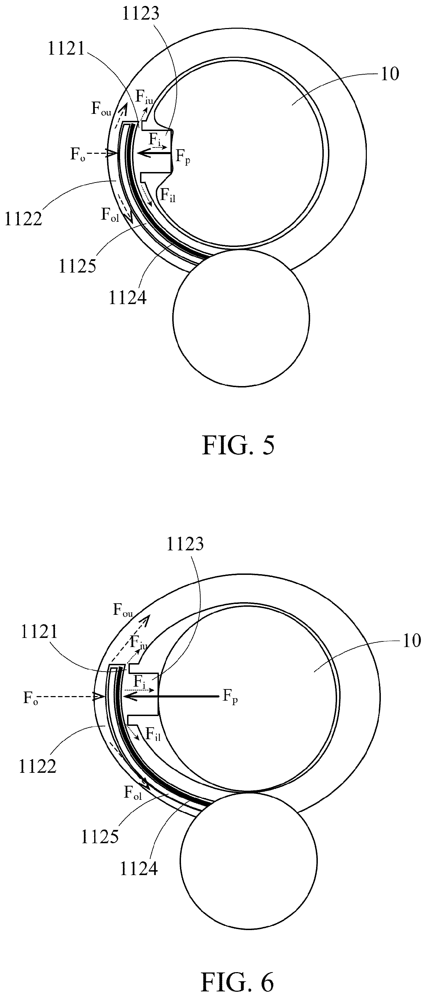

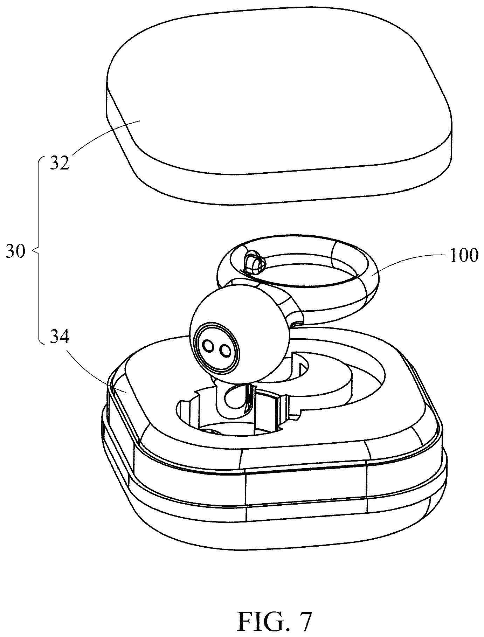

Please refer to FIG. 1 to FIG. 7, FIG. 1 is a schematic front view of a wearable ring-shaped penile erection hardness measuring device according to an embodiment of the present disclosure, FIG. 2 is a schematic cross-section view of the embodiment of FIG. 1, FIG. 3 is a schematic perspective view of the embodiment of FIG. 1, FIG. 4 is a schematic connection diagram of the electronic components in the embodiment of FIG. 1, FIG. 5 shows the measurement principle of the embodiment of FIG. 1 at non-erection state, FIG. 6 shows the measurement principle of the embodiment of the FIG. 1 at erection state, FIG. 7 is a schematic diagram of the combination of the embodiment of the FIG. 1 and a charging device. An embodiment of the present disclosure provides a wearable ring-shaped penile erection hardness measuring device 100 for measuring a hardness value of a penis to be tested 10, the wearable ring-shaped penile erection hardness measuring device 100 includes: an elastic ring-shaped body 110, an electronic component accommodating portion 120, a processing unit 130, and a light emitting element 140.

The elastic ring-shaped body 110 is configured to be worn on the penis to be tested 10, the elastic ring-shaped body 110 includes: a sensing portion 112, and a non-sensing portion 114, wherein the non-sensing portion 114 is connected to the sensing portion 112.

The sensing portion 112 includes an inner layer 1121, an outer layer 1122, a sensing protrusion 1123, a thin film sensing element 1124, a spacer 1125, and an accommodating space 1126. The accommodating space 1126 is located between the inner layer 1121 and the outer layer 1122. The sensing protrusion 1123 is configured to contact the penis to be tested 10. The inner layer 1121 is connected to the sensing protrusion 1123. The thin film sensing element 1124 is disposed in the accommodating space 1126, and the thin film sensing element 1124 contacts the inner layer 1121, wherein the thin film sensing element 1124 is configured to measure an applying force value Fₚ from the penis to be tested 10. The spacer 1125 is disposed in the accommodating space 1126, and the spacer 1125 is located between the thin film sensing element 1124 and the outer layer 1122. In addition, an outer layer thickness Tₒ of the outer layer 1122 is greater than an inner layer thickness Tᵢ of the inner layer 1121.

Specifically, the outer layer thickness Tₒ is between 1.5 mm and 3 mm, the inner layer thickness Tᵢ is between 0.5 mm and 1.2 mm, and a thickness of the accommodating space 1126 is between 0.1 mm and 3 mm. In one example, the outer layer thickness Tₒ is 2.5 mm, the inner layer thickness Tᵢ is 0.9 mm, and the thickness of the accommodating space 1126 is 2 mm. The sensing protrusion 1123 has a protrusion thickness Tₚ, and the protrusion thickness Tₚ is between 1.5 mm and 3 mm. For example, the protrusion thickness Tₚ is 2 mm. The thin film sensing element 1124 is a pressure sensitive element, wherein the pressure sensitive element is a sensing element sensitive to pressure changes, such as a pressure sensitive resistor or the other element with similar characteristics. An inner diameter D of the elastic ring-shaped body 110 is between 2.2 mm and 5.2 mm. It should be understood that the inner diameter D may change according to the need of the user. In addition, the elastic ring-shaped body 110 is made of an elastic material. Specifically, a Shore hardness value of the elastic material is between 20 and 50. For example, the elastic material may be silicone or the other similar materials. It should be noted that the elastic ring-shaped body 110 is integrally formed, i.e. the inner layer 1121, the outer layer 1122, the sensing protrusion 1123 and the non-sensing portion 114 are integrally formed. The inner layer 1121, the outer layer 1122, the sensing protrusion 1123 and the non-sensing portion 114 can be made of the same elastic material (such as, silicone). The spacer 1125 may also be made of an elastic material with a Shore hardness value between 20 and 50.

The electronic component accommodating portion 120 is connected to the elastic ring-shaped body 110. The electronic component accommodating portion 120 is configured to accommodate various electronic components. The electronic component accommodating portion 120 further includes a spherical shell 122, and the spherical shell 122 is used to protect the electronic components disposed in the electronic component accommodating portion. In one embodiment, the electronic component accommodating portion 120 is used to accommodate the processing unit 130, a battery unit 150, a gyroscope 160, a data transmission unit 170 or the other required electronic components.

The processing unit 130 is disposed in the electronic component accommodating portion 120, and the processing unit 130 is electrically connected to the thin film sensing element 1124 to convert the applying force Fₚ into the hardness value.

The light emitting element 140 is adjacent to the electronic component accommodating portion 120, wherein the light emitting element 140 is configured to provide a light with a certain wavelength to the penis to be tested 10. The light may be an ultraviolet ray, a visible ray, or an infrared ray. Specifically, the light with different wavelength may improve the performance of the penis to be tested 10. Taking the light is the infrared ray as an example, the light emitting element 140 is beneficial to dilate the blood vessels of the penis to be tested 10. For example, the certain wavelength of the light emitted by the light emitting element 140 may be 530 nm, 670 nm, or 940 nm. It should be noted that the light emitting element 140 may respectively emit light with the certain wavelength of 530 nm, 670 nm, or 940 nm according to requirements. For example, the penis to be tested 10 is irradiated by the light with a wavelength of 940 nm, which can promote blood vessel dilation and blood circulation, thereby allowing more oxygen to be delivered to the muscle tissue of the penis to be tested 10. For example, the irradiation of light with a wavelength of 670 nm can promote blood vessel dilation, thereby relieving muscle fatigue of the penis to be tested 10. In addition, a portion of the light emitting element 140 may be accommodated in the electronic component accommodating portion 120. In some examples, the light emitting element 140 may include light-emitting diodes (LEDs) or elements with similar function.

The battery unit 150 is accommodated in the electronic component accommodating portion 120 to provide the power required for operating the wearable ring-shaped penile erection hardness measuring device 100. In some examples, the battery unit 150 may be a rechargeable battery.

As shown in FIG. 4, the battery unit 150 is electrically connected to the thin film sensing element 1124, the processing unit 130, the light emitting element 140, the gyroscope 160, and the data transmission unit 170 to provide the power required for the operation of the thin film sensing element 1124, the processing unit 130, the light emitting element 140, the gyroscope 160, and the data transmission unit 170. It should be understood that the battery unit 150 can receive external power for charging through a charging port 152. The processing unit 130 is electrically connected to the thin film sensing element 1124, the light emitting element 140, the gyroscope 160, and the data transmission unit 170. The processing unit 130 not only can receive data from the thin film sensing element 1124, the gyroscope 160, and the data transmission unit 170, but also can transmit data or instructions to the light emitting element 140 and the data transmission unit 170. The gyroscope 160 is used to measure the actual movement data of the penis to be tested 10 during the actual sexual activity. The data transmission unit 170 can transmit data from the processing unit 130 to an external device 20, and the data transmission unit 170 also can transmit data or instructions from the external device 20 to the processing unit 130. Specifically, the data transmission unit 170 may transmit data through wired transmission technology or wireless transmission technology (such as, Bluetooth, WiFi). The external device 20 may be a mobile device (such as, smart phone, tablet), laptop, personal computer or other devices with similar functions.

In addition, as shown in FIG. 7, the wearable ring-shaped penile erection hardness measuring device 100 can be placed in a charging box 30, so as to supply the power of the battery unit 150. The charging box 30 includes a cover 32 and a charging base 34. The wearable ring-shaped penile erection hardness measuring device 100 can be placed in the charging base 34, and the cover 32 can cover the charging base 34 to protect the wearable ring-shaped penile erection hardness measuring device 100 during charging. It should be understood that the charging box 30 may be formed in other feasible structure designs.

Herein, taking the thin film sensing element 1124 as a pressure sensitive resistor as an example, and the measurement principle of the thin film sensing element 1124 is explained with reference to FIGS. 5 and 6. Both the inside surface and the outside surface of the thin film sensing element 1124 (the pressure sensitive resistor) may be used to sense force, wherein the resistance value of the pressure sensitive resistor will reduce in response to the force increases, and the force can be measured by the change in resistance value. Since the inside surface of the thin film sensing element 1124 is in contact with the inner layer 1121, an inside applying force Fᵢ of the inner layer 1121 and a penile pushing force from the sensing protrusion 1123 (i.e. the applying force value Fₚ) will be transferred to the inside surface of the thin film sensing element 1124. Similarly, because the outside surface of the thin film sensing element 1124 is in contact with the spacer 1125 and the spacer 1125 is in contact with the outer layer 1122, an outside applying force Fₒ of the outer layer 1122 will be transferred to the outside surface of the thin film sensing element 1124 through the spacer 1125. The applying force value Fₚ from the penis to be tested can be calculated through the inside applying force Fᵢ, the outside applying force Fₒ, a measured force Fₘ measured by the thin film sensing element 1124, and the following equations: ${{F}_{ou}}^{⇀} + {{F}_{ol}}^{⇀} = {{F}_{o}}^{⇀}$ ${{F}_{ιu}}^{⇀} + {{F}_{ιl}}^{⇀} = {{F}_{ι}}^{⇀}$ $\left|{{F}_{p}}^{⇀}-{{F}_{ι}}^{⇀}\right| = \left|{{F}_{o}}^{⇀}\right| = {F}_{m}$ wherein Fₒᵤ is an applying force of the upper side of the outer layer, Fₒₗ is an applying force of the lower side of the outer layer, Fᵢᵤ is an applying force of the upper side of the inner layer, and Fᵢₗ is an applying force of the lower side of the inner layer. As shown in Equation (1), the outside applying force Fₒ is the resultant force of the applying force of the upper side of the outer layer Fₒᵤ and the applying force of the lower side of the outer layer Fₒₗ. As shown in Equation (2), the inside applying force Fᵢ is the resultant force of the applying force of the upper side of the inner layer Fᵢᵤ and the applying force of the lower side of the inner layer Fᵢₗ. When the penis to be tested 10 contacts the sensing protrusion 1123 and applies the applying force value Fₚ, the inner layer 1121 will generate a smaller force which is opposite to the applying force value Fₚ (i.e. the inside applying force), so that the force, which actually applied on the inside surface of the thin film sensing element 1124, is the applying force value Fₚ minus the inside applying force Fᵢ. Therefore, when the forces are balanced, the outside applying force Fₒ is equal to the applying force value Fₚ minus the inside applying force Fᵢ, and the measured force Fm measured by the thin film sensing element 1124 is the value that the thin film sensing element 1124 at a balance state, as shown in Equation (3). The applying force value Fₚ from the penis to be tested 10 can be calculated through Equation (3).

As shown in FIG. 5, when the penis to be tested 10 is in the non-erection state, the applying force value Fₚ is relatively small. At this time, the outside applying force Fₒ of the outer layer 1122 and the inside applying force Fᵢ of the inner layer 1121 are also relatively small. However, as shown in FIG.6, when the penis to be tested 10 is in the erection state, the applying force value Fₚ from the penis to be tested 10 will increase, and at this time, the outer layer 1122 will extend thereby providing a greater outside applying force Fₒ (relative to the non-erection state). Keeping the outer layer thickness Tₒ of the outer layer 1122 greater than the inner layer thickness Tᵢ of the inner layer 1121 can make the outside applying force Fₒ greater than the inside applying force Fᵢ. In this way, when user only need to know the measured force Fₘ to obtain the applying force value Fₚ, and then the user can convert the applying force value Fₚ into the hardness value. In some embodiments, a plurality of simulated penises with different Shore hardness values may be measured, to obtain a plurality of simulated applying forces corresponding to the simulated penises. The wearable ring-shaped penile erection hardness measuring device 100 can be worn in the penis to be tested 10 to measure the applying force value Fₚ of the penis to be tested 10. Comparing the applying force value with the simulated applying forces, wherein when the applying force value matches one of the simulated applying forces, the Shore hardness value corresponding to the simulated applying force is considered as the hardness value of the penis to be tested 10. In some embodiments, the above-mentioned simulated penises with different shore hardness values and the respective simulated applying forces can be stored in the processing unit 130. In this way, the wearable ring-shaped penile erection hardness measuring device 100 can obtain the hardness value of the penises to be tested 10. In other embodiments, the simulated penises with different Shore hardness values and the respective simulated applying forces can be stored in an external database, and the applying force value measured by the wearable ring-shaped penile erection hardness measuring device 100 is transmitted to the external database through the date transmission unit 170 to comparison for determining the hardness value of the penis to be tested 10.

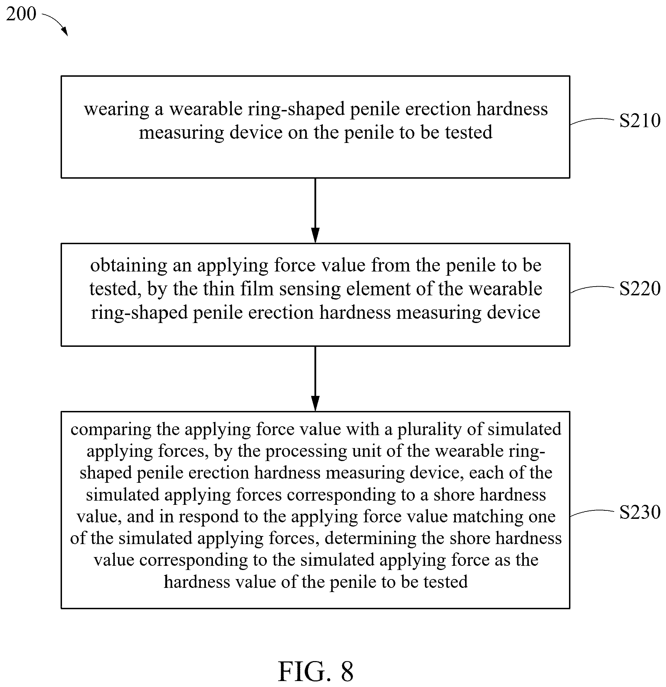

Please refer to FIG. 8, FIG. 8 is a flow chart of a method of measuring penile erection hardness according to an embodiment of the present disclosure. An embodiment of the present disclosure provides a method of measuring penile erection hardness 200 for measuring a hardness value of a penis to be tested. The method of measuring penile erection hardness 200 includes the following steps:

Step S210, wearing a wearable ring-shaped penile erection hardness measuring device on the penis to be tested.

Step S220, obtaining an applying force value from the penis to be tested, by the thin film sensing element of the wearable ring-shaped penile erection hardness measuring device.

Step S230, comparing the applying force value with a plurality of simulated applying forces, by the processing unit of the wearable ring-shaped penile erection hardness measuring device, each of the simulated applying forces corresponding to a Shore hardness value, and in response to the applying force value matching one of the simulated applying forces, determining the Shore hardness value corresponding to the simulated applying force as the hardness value of the penis to be tested. In addition, a wearable ring-shaped penile erection hardness measuring device can be worn on a plurality of simulated penises with different Shore hardness values to obtain a plurality of simulated applying forces. These simulated applying forces and the corresponding Shore hardness values can be stored in the processing unit, and the processing unit can directly compare the applying force with the simulated applying forces to obtain the hardness value of the penis to be tested. Alternatively, these simulated applying forces and the corresponding Shore hardness values can be stored in the external database, the processing unit compares the simulated applying forces stored in the external database, to obtain the hardness value of the penis to be tested.

As described above, the present disclosure transmits the applying force from the penis to be tested to the thin film sensing element through the sensing protrusion, the inner layer, and the outer layer, such that the thin film sensing element can accurately obtain the applying force value from the penis to be tested, and then the applying force value can be converted into the hardness value of the penis to be tested by the processing unit. Measuring the applying force, by the thin film sensing element, can simplify the structure of the wearable ring-shaped penile erection hardness measuring device, thereby reducing the weight of the wearable ring-shaped penile erection hardness measuring device.

In view of the above, although the present invention has been disclosed by way of preferred embodiments, the above preferred embodiments are not intended to limit the present invention, and one of ordinary skill in the art, without departing from the spirit and scope of the invention, the scope of protection of the present invention is defined by the scope of the claims.

## Claims

1. A wearable ring-shaped penile erection hardness measuring device for measuring a hardness value of a penis to be tested, the wearable ring-shaped penile erection hardness measuring device comprising:
an elastic ring-shaped body (110) configured to be worn on the penis to be tested (10), the elastic ring-shaped body (110) comprising:
a sensing portion (112), the sensing portion (112) comprising: an inner layer (1121), an outer layer (1122), a sensing protrusion (1123), a thin film sensing element (1124), and an accommodating space (1126), wherein the accommodating space (1126) is located between the inner layer (1121) and the outer layer (1122), the sensing protrusion (1123) is configured to contact the penis to be tested (10), the inner layer (1121) is connected to the sensing protrusion (1123), the thin film sensing element (1124) is disposed in the accommodating space (1126), the thin film sensing element (1124) contacts the inner layer (1121), and the thin film sensing element (1124) is configured to measure an applying force value (Fₚ) from the penis to be tested (10), wherein an outer layer thickness (Tₒ) of the outer layer (1122) is greater than an inner layer thickness (Tᵢ) of the inner layer (1121); and a non-sensing portion (114) connected to the sensing portion (112);
an electronic component accommodating portion (120) connected to the elastic ring-shaped body (110); and
a processing unit (130) electrically connected to the thin film sensing element (1124) to convert the applying force value (Fₚ) into the hardness value.

2. The wearable ring-shaped penile erection hardness measuring device according to claim 1, **characterized in that** the sensing portion (112) further comprises a spacer (1125) disposed in the accommodating space (1126), and the spacer (1125) is located between the thin film sensing element (1124) and the outer layer (1122).

3. The wearable ring-shaped penile erection hardness measuring device according to claim 3, **characterized in that** the outer layer thickness (To) is between 1.5 mm and 3 mm, and the inner layer thickness (Tᵢ) is between 0.5 mm and 1.2 mm.

4. The wearable ring-shaped penile erection hardness measuring device according to claim 1, **characterized in that** the thin film sensing element (1124) is a pressure sensitive element.

5. The wearable ring-shaped penile erection hardness measuring device according to claim 1, **characterized in that** an inner diameter (D) of the elastic ring-shaped body (110) is between 2.2 mm and 5.2 mm.

6. The wearable ring-shaped penile erection hardness measuring device according to claim 1, further comprising: a light emitting element (140) adjacent to the electronic component accommodating portion (120), **characterized in that** the light emitting element (140) is configured to provide a light with a certain wavelength to the penis to be tested (10).

7. The wearable ring-shaped penile erection hardness measuring device according to claim 1, **characterized in that** the elastic ring-shaped body (110) is made of an elastic material.

8. The wearable ring-shaped penile erection hardness measuring device according to claim 7, **characterized in that** a Shore hardness value of the elastic material is between 20 and 50.

9. The wearable ring-shaped penile erection hardness measuring device according to claim 7, **characterized in that** the elastic material is made of a silicone.

10. A method of measuring penile erection hardness for measuring a hardness value of a penis to be tested (10), the method of measuring penile erection hardness comprising:
wearing a wearable ring-shaped penile erection hardness measuring device of (100) claim 1 on the penis to be tested (10);
obtaining an applying force value (Fₚ) from the penis to be tested (10), by the thin film sensing element (1124) of the wearable ring-shaped penile erection hardness measuring device; and
comparing the applying force value (Fₚ) with a plurality of simulated applying forces, by the processing unit (130) of the wearable ring-shaped penile erection hardness measuring device, each of the simulated applying forces corresponding to a Shore hardness value, and in response to the applying force value (Fₚ) matching one of the simulated applying forces, determining the Shore hardness value corresponding to the simulated applying force as the hardness value of the penis to be tested (10).

## Patentansprüche

1. Eine am Körper tragbare ringförmige Peniserektionshärte-Messvorrichtung zum Messen eines Härtewerts eines zu prüfenden Penis, wobei die am Körper tragbare ringförmige Peniserektionshärte-Messvorrichtung Folgendes beinhaltet:
einen elastischen ringförmigen Körper (110), der konfiguriert ist, um an dem zu prüfenden Penis (10) getragen zu werden, wobei der elastische ringförmige Körper (110) Folgendes beinhaltet:
einen Erfassungsabschnitt (112), wobei der Erfassungsabschnitt (112) Folgendes beinhaltet: eine Innenschicht (1121), eine Außenschicht (1122), einen Erfassungsvorsprung (1123), ein Dünnfilm-Erfassungselement (1124) und einen Aufnahmeraum (1126), wobei sich der Aufnahmeraum (1126) zwischen der Innenschicht (1121) und der Außenschicht (1122) befindet, der Erfassungsvorsprung (1123) konfiguriert ist, um den zu prüfenden Penis (10) zu berühren, die Innenschicht (1121) mit dem Erfassungsvorsprung (1123) verbunden ist, das Dünnfilm-Erfassungselement (1124) in dem Aufnahmeraum (1126) angeordnet ist, das Dünnfilm-Erfassungselement (1124) die Innenschicht (1121) berührt und das Dünnfilm-Erfassungselement (1124) konfiguriert ist, um einen Aufbringungskraftwert (Fₚ) von dem zu prüfenden Penis (10) zu messen, wobei eine Außenschichtdicke (Tₒ) der Außenschicht (1122) größer als eine Innenschichtdicke (Tᵢ) der Innenschicht (1121) ist; und
einen Nicht-Erfassungsabschnitt (114), der mit dem Erfassungsabschnitt (112) verbunden ist;
einen Elektronikkomponenten-Aufnahmeabschnitt (120), der mit dem elastischen ringförmigen Körper (110) verbunden ist; und
eine Verarbeitungseinheit (130), die mit dem Dünnfilm-Erfassungselement (1124) elektrisch verbunden ist, um den Aufbringungskraftwert (Fₚ) in den Härtewert umzuwandeln.

2. Am Körper tragbare ringförmige Peniserektionshärte-Messvorrichtung gemäß Anspruch 1, **dadurch gekennzeichnet, dass** der Erfassungsabschnitt (112) ferner einen Abstandhalter (1125) beinhaltet, der in dem Aufnahmeraum (1126) angeordnet ist, und der Abstandhalter (1125) sich zwischen dem Dünnfilm-Erfassungselement (1124) und der Außenschicht (1122) befindet.

3. Am Körper tragbare ringförmige Peniserektionshärte-Messvorrichtung gemäß Anspruch 3, **dadurch gekennzeichnet, dass** die Außenschichtdicke (Tₒ) zwischen 1,5 mm und 3 mm beträgt und die Innenschichtdicke (Tᵢ) zwischen 0,5 mm und 1,2 mm beträgt.

4. Am Körper tragbare ringförmige Peniserektionshärte-Messvorrichtung gemäß Anspruch 1, **dadurch gekennzeichnet, dass** das Dünnfilm-Erfassungselement (1124) ein druckempfindliches Element ist.

5. Am Körper tragbare ringförmige Peniserektionshärte-Messvorrichtung gemäß Anspruch 1, **dadurch gekennzeichnet, dass** ein Innendurchmesser (D) des elastischen ringförmigen Körpers (110) zwischen 2.2 mm und 5.2 mm beträgt.

6. Am Körper tragbare ringförmige Peniserektionshärte-Messvorrichtung gemäß Anspruch 1, die ferner Folgendes beinhaltet: ein lichtemittierendes Element (140), das an den Elektronikkomponenten-Aufnahmeabschnitt (120) angrenzt, **dadurch gekennzeichnet, dass** das lichtemittierende Element (140) konfiguriert ist, um dem zu prüfenden Penis (10) ein Licht mit einer gewissen Wellenlänge bereitzustellen.

7. Am Körper tragbare ringförmige Peniserektionshärte-Messvorrichtung gemäß Anspruch 1, **dadurch gekennzeichnet, dass** der elastische ringförmige Körper (110) aus einem elastischen Material hergestellt ist.

8. Am Körper tragbare ringförmige Peniserektionshärte-Messvorrichtung gemäß Anspruch 7, **dadurch gekennzeichnet, dass** ein Shore-Härtewert des elastischen Materials zwischen 20 und 50 beträgt.

9. Am Körper tragbare ringförmige Peniserektionshärte-Messvorrichtung gemäß Anspruch 7, **dadurch gekennzeichnet, dass** das elastische Material aus Silikon hergestellt ist.

10. Ein Verfahren zum Messen einer Peniserektionshärte zum Messen eines Härtewerts eines zu prüfenden Penis (10), wobei das Verfahren zum Messen einer Peniserektionshärte Folgendes beinhaltet:
Tragen einer am Körper tragbaren ringförmigen Peniserektionshärte-Messvorrichtung (100) gemäß Anspruch 1 auf dem zu prüfenden Penis (10);
Erhalten eines Aufbringungskraftwerts (Fₚ) von dem zu prüfenden Penis (10) durch das Dünnfilm-Erfassungselement (1124) der am Körper tragbaren ringförmigen Peniserektionshärte-Messvorrichtung; und
Vergleichen des Aufbringungskraftwerts (Fₚ) mit einer Vielzahl von simulierten Aufbringungskräften, durch die Verarbeitungseinheit (130) der am Körper tragbaren ringförmigen Peniserektionshärte-Messvorrichtung, wobei jede der simulierten Aufbringungskräfte einem Shore-Härtewert entspricht, und als Reaktion darauf, dass ein Aufbringungskraftwert (Fₚ) mit einer der simulierten Aufbringungskräfte übereinstimmt, Bestimmen des Shore-Härtewerts, der der simulierten Aufbrinungskraft entspricht, als Härtewert des zu prüfenden Penis (10).

## Revendications

1. Un dispositif de mesure de dureté d'érection pénienne en forme d'anneau pouvant être porté pour la mesure d'une valeur de dureté d'un pénis devant être testé, le dispositif de mesure de dureté d'érection pénienne en forme d'anneau pouvant être porté comprenant :
un corps élastique en forme d'anneau (110) configuré pour être porté sur le pénis devant être testé (10), le corps élastique en forme d'anneau (110) comprenant :
une portion de détection (112), la portion de détection (112) comprenant : une couche interne (1121), une couche externe (1122), une saillie de détection (1123), un élément de détection à film mince (1124), et un espace contenant (1126), l'espace contenant (1126) étant situé entre la couche interne (1121) et la couche externe (1122), la saillie de détection (1123) étant configurée pour se mettre au contact du pénis devant être testé (10), la couche interne (1121) étant reliée à la saillie de détection (1123), l'élément de détection à film mince (1124) étant disposé dans l'espace contenant (1126), l'élément de détection à film mince (1124) étant au contact de la couche interne (1121), et l'élément de détection à film mince (1124) étant configuré pour mesurer une valeur de force d'application (Fₚ) en provenance du pénis devant être testé (10), où une épaisseur de couche externe (Tₒ) de la couche externe (1122) est plus importante qu'une épaisseur de couche interne (Tᵢ) de la couche interne (1121) ; et
une portion de non-détection (114) reliée à la portion de détection (112) ;
une portion contenant des composants électroniques (120) reliée au corps élastique en forme d'anneau (110) ; et
une unité de traitement (130) reliée électriquement à l'élément de détection à film mince (1124) pour convertir la valeur de force d'application (Fₚ) en valeur de dureté.

2. Le dispositif de mesure de dureté d'érection pénienne en forme d'anneau pouvant être porté selon la revendication 1, **caractérisé en ce que** la portion de détection (112) comprend en outre un espaceur (1125) disposé dans l'espace contenant (1126), et l'espaceur (1125) est situé entre l'élément de détection à film mince (1124) et la couche externe (1122).

3. Le dispositif de mesure de dureté d'érection pénienne en forme d'anneau pouvant être porté selon la revendication 3, **caractérisé en ce que** l'épaisseur de couche externe (Tₒ) est entre 1,5 mm et 3 mm, et l'épaisseur de couche interne (Tᵢ) est entre 0,5 mm et 1,2 mm.

4. Le dispositif de mesure de dureté d'érection pénienne en forme d'anneau pouvant être porté selon la revendication 1, **caractérisé en ce que** l'élément de détection à film mince (1124) est un élément sensible à la pression.

5. Le dispositif de mesure de dureté d'érection pénienne en forme d'anneau pouvant être porté selon la revendication 1, **caractérisé en ce qu'**un diamètre interne (D) du corps élastique en forme d'anneau (110) est entre 2,2 mm et 5,2 mm.

6. Le dispositif de mesure de dureté d'érection pénienne en forme d'anneau pouvant être porté selon la revendication 1, comprenant en outre : un élément émettant de la lumière (140) adjacent à la portion contenant des composants électroniques (120), **caractérisé en ce que** l'élément émettant de la lumière (140) est configuré pour fournir une lumière ayant une certaine longueur d'onde au pénis devant être testé (10).

7. Le dispositif de mesure de dureté d'érection pénienne en forme d'anneau pouvant être porté selon la revendication 1, **caractérisé en ce que** le corps élastique en forme d'anneau (110) est fait d'un matériau élastique.

8. Le dispositif de mesure de dureté d'érection pénienne en forme d'anneau pouvant être porté selon la revendication 7, **caractérisé en ce qu'**une valeur de dureté Shore du matériau élastique est entre 20 et 50.

9. Le dispositif de mesure de dureté d'érection pénienne en forme d'anneau pouvant être porté selon la revendication 7, **caractérisé en ce que** le matériau élastique est fait d'une silicone.

10. Un procédé de mesure de dureté d'érection pénienne pour la mesure d'une valeur de dureté d'un pénis devant être testé (10), le procédé de mesure de dureté d'érection pénienne comprenant :
le port d'un dispositif de mesure de dureté d'érection pénienne en forme d'anneau pouvant être porté (100) de la revendication 1 sur le pénis devant être testé (10) ;
l'obtention d'une valeur de force d'application (Fₚ) en provenance du pénis devant être testé (10), par l'élément de détection à film mince (1124) du dispositif de mesure de dureté d'érection pénienne en forme d'anneau pouvant être porté ; et
la comparaison de la valeur de force d'application (Fₚ) avec une pluralité de forces d'application simulées, par l'unité de traitement (130) du dispositif de mesure de dureté d'érection pénienne en forme d'anneau pouvant être porté, chacune des forces d'application simulées correspondant à une valeur de dureté Shore, et en réponse au fait que la valeur de force d'application (Fₚ) concorde avec l'une des forces d'application simulées, la détermination de la valeur de dureté Shore correspondant à la force d'application simulée en tant que valeur de dureté du pénis devant être testé (10).
